# EUROPÄISCHE PATENTSCHRIFT

(11) **EP 0 967 015 B1**
(45) Veröffentlichungstag und Bekanntmachung des Hinweises auf die Patenterteilung: **12.01.2005**
(21) Anmeldenummer: 99111459.6
(22) Anmeldetag: 12.06.1999
(51) Int. Cl.: B01J 31/22, C07B 53/00, C07C 227/32

(54) **Verwendung von Ferrocenylliganden zur katalytischen enantioselektiven Hydrierung**
Use of ferrocenyl ligands in catalytic enantioselective hydrogenation
Utilisation de ligands ferrocenyl pour l'hydrogénation catalytique énantioselective

(30) Priorität: 19.06.1998 DE 19827311; 12.05.1999 DE 19921924
(43) Veröffentlichungstag der Anmeldung: 29.12.1999
(73) Patentinhaber: Degussa AG, 40474 Düsseldorf (DE)
(72) Erfinder: Knochel, Paul Prof., 35037 Marburg (DE); Almena Perea, Juan José Dr., 63450 Hanau (DE); Drauz, Karlheinz Prof., 63579 Freigericht (DE); Klement, Ingo Dr., 35415 Pohlheim-Grabenteich (DE)

(56) Entgegenhaltungen:
- US-A- 5 434 269
- US-A- 5 596 114
- JAHYO K ET AL: "Asymmetric Synthesis of A New Cylindrically Chiral and Air-Stable Ferrocenyldiphosphine and Its Application to Rhodium-Catalyzed Asymmetric Hydrogenation" TETRAHEDRON LETTERS,NL,ELSEVIER SCIENCE PUBLISHERS, AMSTERDAM, Bd. 39, Nr. 31, 30. Juli 1998 (1998-07-30), Seiten 5523-5526, XP004124106 ISSN: 0040-4039
- SCHWINK L ET AL: "New C2-symmetrical ferrocenyl diamines as ligands for ruthenium catalyzed transfer hydrogenation" TETRAHEDRON: ASYMMETRY,NL,ELSEVIER SCIENCE PUBLISHERS, AMSTERDAM, Bd. 9, Nr. 7, 9. April 1998 (1998-04-09), Seiten 1143-1163, XP004116315 ISSN: 0957-4166

## Beschreibung

Die vorliegende Erfindung betrifft die Verwendung von enantiomerenangereicherten Liganden der allgemeinen Formel I und deren Salze 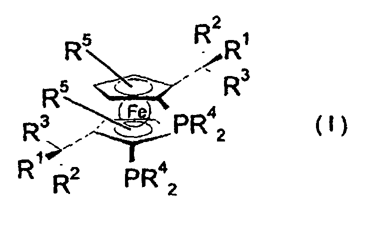 worin
R¹, R² unabhängig voneinander bedeuten H, N(C₁-C₈)-Alkyl₂, NH(C₁-C₈)-Acyl, N(C₁-C₈)-Acyl₂, O(C₁-C₈)-Acyl, (C₁-C₈)-Alkyl, (C₁-C₈)-Alkoxy, (C₂-C₈)-Alkoxyalkyl, welche gegebenenfalls linear oder verzweigt vorliegen, (C₃-C₇)-Cycloalkyl (C₆-C₁₈)-Aryl, wie Phenyl; 1-, 2-Naphthyl oder Anthryl,
R³ bedeutet (C₃-C₇)-Cycloalkyl, (C₆-C₁₈)-Aryl, wie Phenyl, 1-, 2-Naphthyl oder Anthryl, wobei die eben genannten Reste gegebenenfalls einfach oder mehrfach mit linear oder verzweigten (C₁-C₈)-Alkyl, mit Halogenen substituiert sein können,
R⁴ bedeutet (C₁-C₈)-Alkyl, (C₃-C₇)-Cycloalkyl, (C₆-C₁₈)-Aryl, wie Phenyl, 1-, 2-Naphthyl oder Anthryl, , wobei die eben genannten Reste gegebenenfalls einfach oder mehrfach mit linear oder verzweigten (C₁-C₈)-Alkyl, mit Halogenen substituiert sein können,
R⁵ bedeutet H, zur homogenen katalytischen enantioselektiven Hydrierung.

Des weiteren richtet'sich die Erfindung auf die Verwendung von enantiomerenangereicherten Komplexe der allgemeinen Formel II und deren Salze 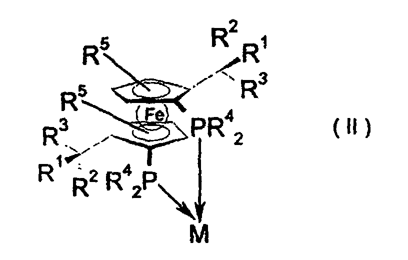 worin
R¹ bis R⁵ die oben angegebene Bedeutung annehmen und M ein Metallatom oder -ion der Nebengruppe 8, zB. Ni, Co, Rh, Ru, Ir, Pd, Re oder Pt ist zur enantioselektiven homogenen katalytischen Hydrierung.

Die homogene katalytische enantioselektive Hydrierung von Iminen und Enaminen besitzt für die Herstellung von z.B. enantiomerenangereicherten Aminosäuren großes Interesse, da diese wiederum als chirale Edukte in der organischen Synthese z.B. von bioaktiven Wirkstoffen benötigt werden.

Der Einsatz von Bisphosphinkatalysatoren für die enantioselektive homogene katalytische Hydrierung für den eben genannten Zweck ist wohl bekannt (Burk et al., Tetrahedron 1994, 4399). Hayashi et al. (J. Chem. Soc., Chem Commun. 1989, 495-496), Knochel et al. (Chem. Eur. J. 1998, 4, 950-968) und Ikeda et al. (Tetrahedron Lett. 1996, 4545-4448) beschreiben Pd-Komplexe mit *C*₂-symmetrische Ferrocenyl-(bis-tertiärphosphin)-Liganden. Allerdings wurden diese Komplexe lediglich bei asymmetrischen Allylierungen bzw. Kreuzkupplungen eingesetzt. Die Verwendung der Liganden bei der enantioselektiven Hydrierung ist bislang nicht bekannt.

Yamamoto et al. (Bull. Chem. Soc. Jpn. 1980, 53, 1132-1137) berichteten über den Einsatz von nicht *C*₂-symmetrischen Ferrocenyl-(bis-tertiär-phosphin)-Liganden in der enantioselektiven homogenen katalytischen Hydrierung. Mit diesen Liganden erhält man jedoch nur sehr vereinzelt gute Enantiomerenüberschüsse.

Aufgabe der vorliegenden Erfindung ist deshalb die Angabe einer Verwendung C₂-symmetrischer enantiomerenangereicherter Bisphosphinligandensysteme und - katalysatoren für die homogene enantioselektive katalytische Hydrierung von Doppel-Bindungen zwischen einem Kohlenstoffatom und einem weiteren Kohlenstoffatom oder Stickstoffatom verstanden.

Dadurch, daß für die homogene katalytische enantioselektive Hydrierung von C=C bzw. C=N- Doppelbindungen enantiomerenangereicherte Liganden der allgemeinen Formel I sowie deren Salze, 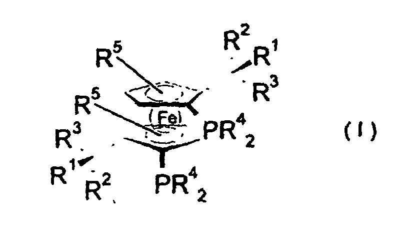 worin
R¹, R² unabhängig voneinander bedeuten H, N(C₁-C₈)-Alkyl₂, NH(C₁-C₈)-Acyl, N(C₁-C₈)-Acyl₂, O(C₁-C₈)-Acyl, (C₁-C₈)-Alkyl, (C₁-C₈)-Alkoxy, (C₂-C₈)-Alkoxyalkyl, welche gegebenenfalls linear oder verzweigt vorliegen, (C₃-C₇)-Cycloalkyl (C₆-C₁₈)-Aryl, wie Phenyl; 1-, 2-Naphthyl oder Anthryl,
R³ bedeutet (C₃-C₇)-Cycloalkyl, (C₆-C₁₈)-Aryl, wie Phenyl, 1-, 2-Naphthyl oder Anthryl, wobei die eben genannten Reste gegebenenfalls einfach oder mehrfach mit linear oder verzweigten (C₁-C₈)-Alkyl, mit Halogenen substituiert sein können,
R⁴ bedeutet (C₁-C₈)-Alkyl, (C₃-C₇)-Cycloalkyl, (C₆-C₁₈)-Aryl, wie Phenyl, 1-, 2-Naphthyl oder Anthryl, wobei die eben genannten Reste gegebenenfalls einfach oder mehrfach mit linear oder verzweigten (C₁-C₈)-Alkyl, mit Halogenen substituiert sein können,
R⁵ bedeutet H,
verwendet werden, erhält man die entsprechend hydrierten Derivate in sehr guten Raum/Zeit-Ausbeuten und mit einem sehr hohen ee-Wert behaftet.

Bevorzugt ist die Verwendung von Liganden der Formel I, worin
R¹, R² unabhängig voneinander bedeuten H, O(C₁-C₈)-Acyl, N(C₁-C₈)-Alkyl₂, (C₁-C₈)-Alkyl
R³ bedeutet (C₆-C₁₈)-Aryl, wie Phenyl, 1-,2-Naphthyl oder Anthryl, wobei die eben genannten Reste gegebenenfalls einfach oder mehrfach mit linear oder verzweigten (C₁-C₈)-Alkyl, mit Halogenen substituiert sein können,
R⁴ bedeutet Phenyl,
R⁵ bedeutet H.

Ein weiterer Aspekt der Erfindung beschäftigt sich mit der Verwendung von enantiomerenangereicherten Komplexen der allgemeinen Formel II und deren Salzen 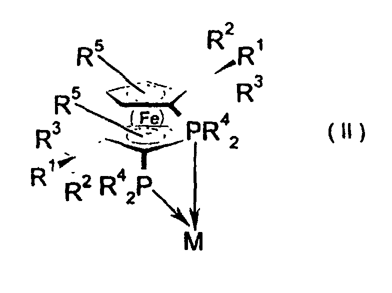 worin
R¹ bis R⁵ die oben angegebene Bedeutung annehmen und M ein Metallatom oder -ion der Nebengruppe 8, z. B. Co, Ni, Rh, Ru, Ir, Pd, Re ist zur homogenen katalytischen enantioselektiven Hydrierung von C=C- bzw. C=N-Doppelbindungen. Die in der oben angegebenen allgemeinen Formel für den erfindungsgemäßen Komplex (II) freigelassenen Koordinationsstellen des Zentralatoms M sind durch dem Fachmann bekannte Liganden für diese Reaktion (R. Schrock, J. A. Osborn J. Am. Chem. Soc. 1971, 93, 2397-2407, R. Glaser, S. Geresh, J. Blumenfeld J. Organomet. Chem. 1976, *112*, 355-360) oder gegebenenfalls das bei der Reaktion vorhandene Lösungsmittel ausgefüllt. Die Koordinatoren sind jeweils zu der entsprechenden Formel (II) hinzuzudenken.

Die erfindungsgemäße Verwendung der Liganden und Komplexe ist vergleichbaren Hydrierungen aus dem Stand der Technik überlegen. So zeigen die Komplexe eine derart hohe Aktivität für die betrachtete Reaktion und eine ausgesprochen geringe Empfindlichkeit gegenüber der Oxidation durch Luftsauerstoff, daß im Gegensatz zu vergleichbaren Katalysatoren des Standes der Technik auf die Entgasung und den Einsatz von p.A. Lösungsmitteln verzichtet werden kann. Es 'reicht aus, Lösungsmittel bei der Hydrierung mit technischer Qualität einzusetzen.

Bevorzugt ist die Verwendung von katalysatoren der Formel II, worin
R¹, R² unabhängig voneinander bedeuten H, O(C₁-C₈)-Acyl, N(C₁-C₈)-Alkyl₂, (C₁-C₈)-Alkyl
R³ bedeutet (C₆-C₁₈)-Aryl, wie Phenyl, 1-,2-Naphthyl oder Anthryl, wobei die eben genannten Reste gegebenenfalls einfach oder mehrfach mit linear oder verzweigten (C₁-C₈)-Alkyl, mit Halogenen substituiert sein können,
R⁴ bedeutet Phenyl,
R⁵ bedeutet H.

Die aus den Ligandensystemen herstellbaren Komplexe zeigen bei der homogenen enantioselektiven katalytischen Hydrierung ausgezeichnete Werte, wie folgende Tabellen belegen.

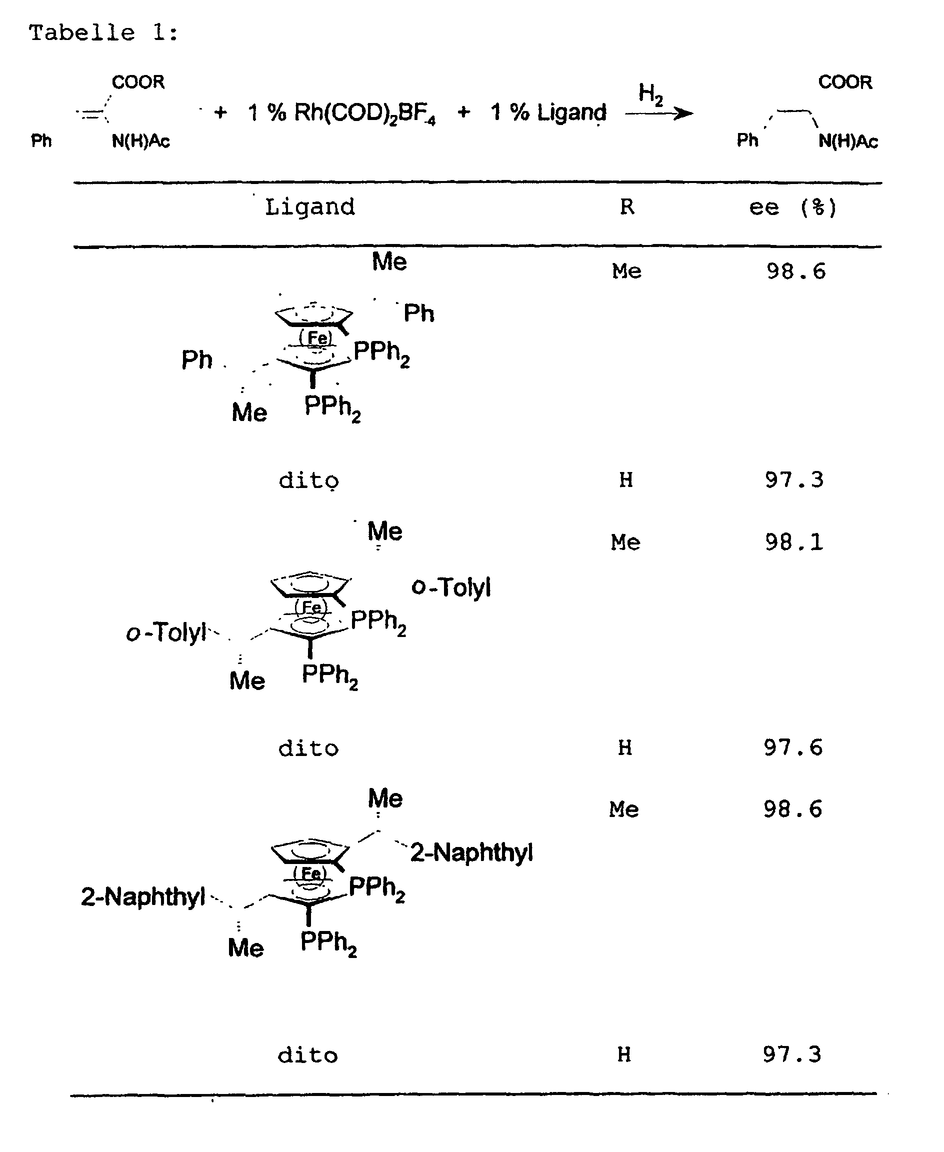

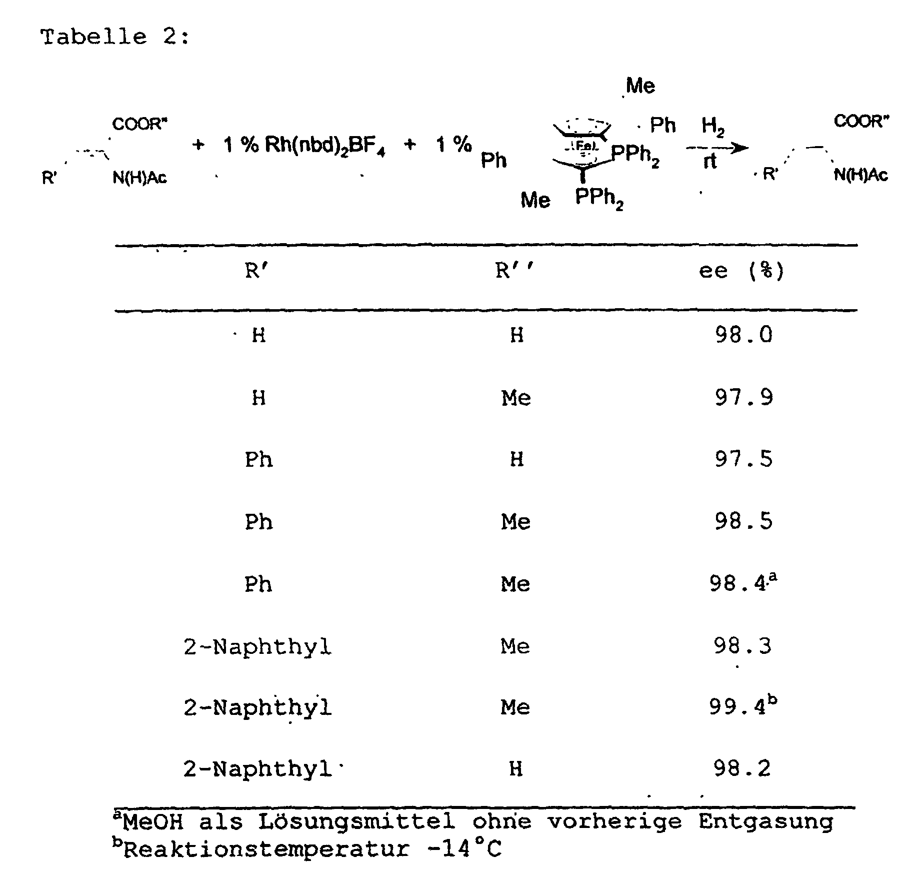

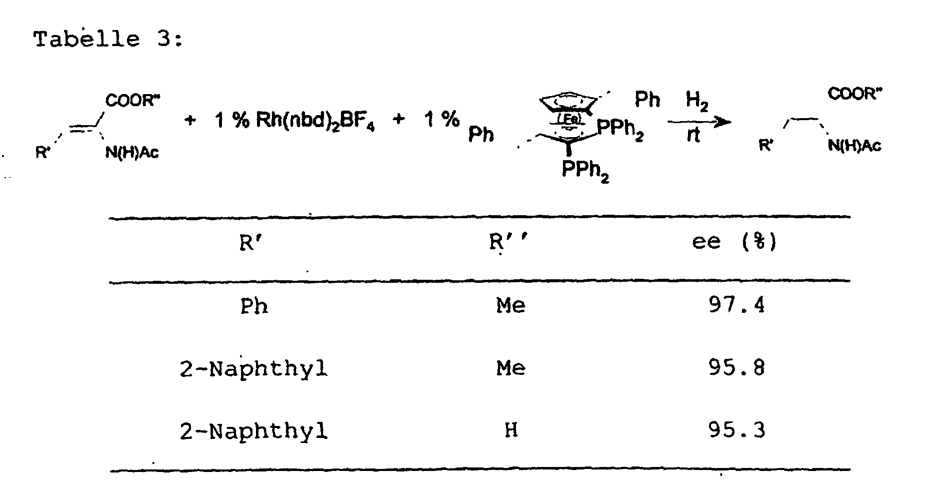

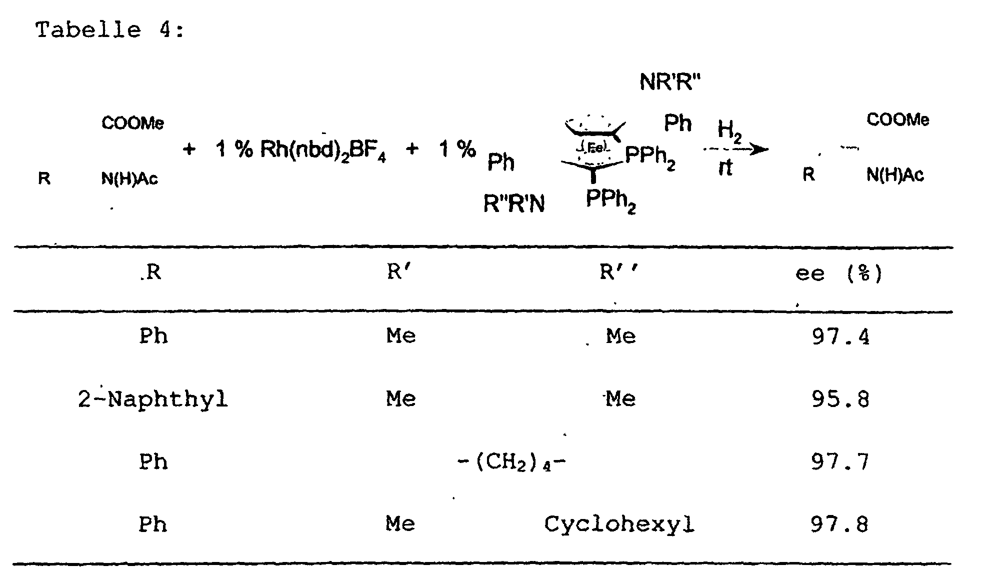

nbd ist die Abkürzung für 2,5-Norbornadien, COD steht für 1,5-Cyclooctadien.

Die'Reaktionsdauer bei oben gezeigten Umsetzungen beträgt <60 min. Die Katalysatorkonzentration ist mit 1% in den genannten Beispielen bereits sehr niedrig. Sie kann jedoch für technische Anwendung beträchtlich erniedrigt werden. Dies beides ist für die Anwendung der erfindungsgemäßen Liganden im technischen Maßstab sehr von Vorteil, da die Kosten für die nach diesem Verfahren gewonnenen Produkte entsprechend niedriger ausfallen und damit ein höherer ökonomischer Nutzen als bei Verwendung von Ligandensystemen/Komplexen des Standes der Technik 'garantiert wird. Dieser Vorteil ist durch die ausgesprochen gute Aktivität der Ligandensysteme bzw. Komplexe bedingt.

Die Ligandensysteme sind darüberhinaus derart oxidationsunempfindlich, daß sie bei Umgebungsbedingungen lange Zeit unverändert haltbar sind. Dies ist für die Lagerung in großem Maßstab ebenfalls von Vorteil.

Die Herstellung der Ligandensysteme ist in dem Stand der Technik beschrieben (Knochel et al., Chem. Eur. J. 1998, 4, 950f.; Enders et al., Syn. Lett. 1997, 355f.; Knochel et al., Tetrahedron Lett. 1996, 37, 25f.; Schmalz et al., Tetrahedron 1997, 53, 7219f.). Eine Übersicht zeigt weitere mögliche Synthesewege auf (Schema 1).

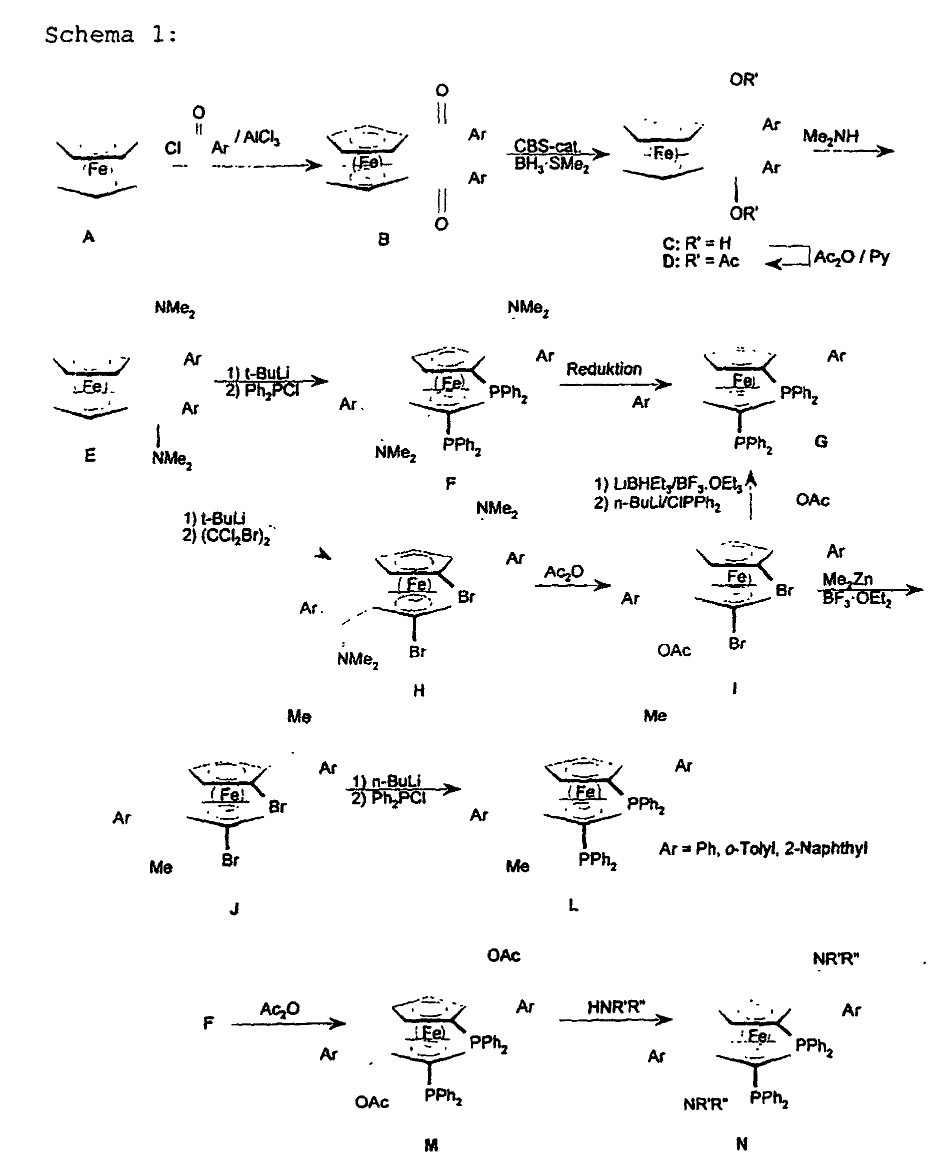

Die Einführung einer bevorzugten zentralen und planaren Chiralität in das 1,1'-diacylierte Ferrocen kann im Prinzip nach allen dem Fachmann für diese Reaktion in Frage kommenden Methoden erfolgen (J. Am Chem. Soc. 1957, 79, 2742, J. Organomet. Chem. 1973, *52*, 407-424). Bevorzugt ist allerdings die Reduktion mit dem sogenannten CBS-Reagenz (J. Am. Chem. Soc. 1987, 109, 5551-5553, Tetrahedron Lett. 1996, *37*, 25-28). Durch diese Maßnahme ist gewährleistet, daß die Reduktionsprodukte in sehr guten Ausbeuten und mit einer sehr hohen optischen und diastereomeren Reinheit anfallen. Ein weiterer denkbarer Weg zur Herstellung gewünschter enantiomerenangereicherter Liganden kann beispielsweise darin gesehen werden, die 1,1'-diacylierten Ferrocene mittels enantioselektiver reduktiver Aminierung herzustellen. Man gelangt so gleich zu den enantiomerenangereicherten Liganden mit einem Amin-Substituenten am stereogenen Zentrum.

Weitere Möglichkeiten zur Einführung der Chiralität werden prinzipiell in Tetrahedron Asymmetry 1991, *2*, 601-612, J. Org. Chem. 1991, 56, 1670-1672, J. Org. Chem. 1994, 59, 7908-7909, J. Chem. Soc., Chem. Commun. 1990, 888-889 beschrieben.

Zur Einführung des Restes R⁵ kann man in einem Deprotonierungsschritt die aciden Protonen am Ring deprotonieren und die deprotonierte Spezies anschließend zur Einführung eines Restes R⁵ mit einem geeigneten elektrophilen Reagenz umsetzen.

Der Rest R⁵ kann u. a. zur Anbindung der erfindungsgemäßen Komplexe an eine polymere Matrix wie z.B. ein lineares PMMA, Polystyrol oder PEG sowie ein nichtlineares Dendrimer benutzt werden. Die Anbindung des Restes R⁵ an den Cyclopentadienylring des erfindungsgemäßen Komplexes ist bzgl. der freien Positionen variabel. Als Reste können alle dem Fachmann für diesen Zweck in Frage kommenden Reste verwandt werden. Eine geeignete Übersicht zur molekularen Vergrößerung von Komplexkatalysatoren bietet (Tetrahedron Asymmetry 1998, *9,* 691-696). Bevorzugt besteht der Rest R⁵ aus.der Anordnung B-X-Z, wobei B ein Rest der Gruppe CR⁸₂, NR⁸, O, S, SiR⁸₂, X ein Spacer, wie z.B. 1,4'-Biphenyl, 1-, 2-Ethylen, 1-, 3-Propylen, PEG-(2-10) und Z ein über eine funktionelle Gruppe, wie z.B. die O-, NH-, COO-, CONH, Ethenyl-, NHCONH-, OCONH- oder NHCOO-Funktion, an ein wie oben geschildertes Polymer gebundener Rest ist. Alternativ können die Reste R⁵ der beiden Cyclopentadienylringe über eine α,ω-(C₂-C₄)-Alkylenbrücke miteinander verbunden sein.

Als linear oder verzweigte (C₁-C₈)- oder (C₂-C₈)-Alkylreste sind anzusehen Methyl, Ethyl, *n*-Propyl, Isopropyl, *n*-Butyl, Isobutyl, *sec*-Butyl, *tert*-Butyl, Pentyl, Hexyl, Heptyl oder Octyl samt aller ihrer Bindungsisomeren. Der Rest (C₁-C₈)-Alkoxy entspricht dem Rest (C₁-C₈)-Alkyl mit der Maßgabe, daß dieser über ein Sauerstoffatom an das Molekül gebunden ist. Als (C₂-C₈)-Alkoxyalkyl sind Reste gemeint, bei denen die Alkylkette durch mindestens eine Sauerstoffunktion unterbrochen ist, wobei nicht zwei Sauerstoffatome miteinander verbunden sein können. Die Anzahl der Kohlenstoffatome gibt die Gesamtzahl der im Rest enthaltenen Kohlenstoffatome an. N-, O-, P-, S-atomhaltige Reste sind insbesondere Alkylreste der oben genannten Art, welche eines oder mehrere dieser Heteroatome in ihrer Kette aufweisen bzw. welche über eines dieser Heteroatome an das Molekül gebunden sind. Unter (C₃-C₇)-Cycloalkyl versteht man Cyclopropyl, Cyclobutyl, Cyclopentyl, Cyclohexyl bzw. Cycloheptylreste.

(C₁-C₈)-Acyloxy bedeutet im Rahmen der Erfindung einen wie oben definierten Alkylrest, welcher über eine COO-Funktion an das Molekül gebunden ist.

(C₁-C₈)-Acyl bedeutet im Rahmen der Erfindung einen wie oben definierten Alkylrest, welcher über eine CO-Funktion an das Molekül gebunden ist.

Als Halogene kommen Fluor, Chlor, Brom und Iod in Frage.

Unter Salzen versteht man ionische Additionsverbindungen aus starken Säuren wie HCl, HBr, H₂SO₄; H₃PO₄, CF₃COOH, p-Toluolsulfonsäure, Methansulfonsäure und dem betrachteten Molekül.

PEG bedeutet Polyethylenglykol.

Die folgenden Beispiele sollen die Erfindung erläutern.

### Beipiele:

### Generelle Vorgehensweise

Alle Reaktionen wurden, falls nicht anders angegeben, unter Argon ausgeführt. Die Aufarbeitung der Reaktionsmischungen erfolgte wie folgt: Hydrolyse mit ges. NH₄Cl-Lsg., Extraktion mit Methyl-tert.-butylether (MTBE) (3 x 10 ml), Waschen der kombinierten org. Extrakte mit ges. NaCl-Lsg. (20 ml), Trocknung über MgSO₄, Filtration, Einengung der Filtrate im Vakuum und Reinigung der Rückstände per Säulenchromatographie mit Kieselgel 60 (70 - 230 mesh ASTM) und unterschiedlichen Zusammensetzungen von Gemischen aus Hexan/MTBE. Schmelzpunkte sind unkorrigiert.

Das verwendete Methanol wurde vorher über Mg getrockent und unter Argon destilliert, sowie mit Argon entgast. HPLC-Methanol wurde ohne vorherige Reinigung eingesetzt. Es hat sich gezeigt, daß HPLC-Methanol auch durch nicht aufgearbeitetes technisches Methanol ersetzt werden kann.

### 1. Allgemeine Herstellvorschrift für 1,1'-Diacylferrocene durch Friedel-Crafts-Acylierung (A->B)

Zu einer Suspension von Aluminium-(III)-chlorid in CH₂Cl₂ (10 ml) bei 0°C wird Acetylchlorid gegeben. Das Ferrocen wird anschließend, in 10 ml CH₂Cl₂ gelöst, zu dieser Mischung tropfenweise binnen 20 min hinzugegeben. Die Reaktionsmischung wird auf Raumtemp. erwärmt und 2 h gerührt. Anschließend wird mit Eiswasser bei 0°C tropfenweise hydrolysiert. Die Mischung wird mit 100 ml Methylenchlorid verdünnt, zweimal mit wäßriger K₂CO₃-Lsg. (50 ml) und dann mit ges. NaCl-Lsg. (50 ml) gewaschen. Die org. Phase wird getrocknet und aufkonzentriert. Der Rückstand wird per Säulenchromatographie gereinigt.

*1,1'-Dibenzoylferrocen:* Aus Ferrocen (13.95 g, 75.0 mmol), Benzoylchlorid (19.2. mL, 165.0 mmol) und Aluminium(III)chlorid (22.00 g, 165.0 mmol) wird der Feststoff in einer Ausbeute von 91 % (27.1 g, 68.3 mmol) nach Kristallisation aus Pentan erhalten; roter Feststoff m.p. 97-100°C (lit. 106.5-106.7 °C); IR (KBr): νₘₐₓ = 3267 (w), 3113 (w), 3064 (w), 1637 (vs), 1448 (s), 1288 (s), 1048 (m), 846 (m), 726 (s), 698 (s).

*1,1'-Di(o-toluoyl)ferrocen:* Aus Ferrocen (1.43 g, 7.7 mmol), *o*-Toluoylchlorid (2.11 mL, 16.2 mmol) und Aluminium(III)chlorid (2.16 g, 16.2 mmol) wird der Feststoff in einer Ausbeute von 73 % (2.36 g, 5.6 mmol) nach säulenchromatographischer Reinigung erhalten; roter Feststoff m.p. 124-125 °C; IR (KBr): νₘₐₓ = 3085 (w), 2923 (w), 1647 (vs), 1443 (m), 1273 (s), 840 (m), 737 (s).

*1,1'-Di(2-naphthoyl)ferrocen:* Aus Ferrocen (1.86 g, 10.0 mmol), 2-Naphthoylchlorid (4.2 g, 22.0 mmol) und Aluminium(III)chlorid (3.5 g, 26.0 mmol) wird nach säulenchromatographischer Reinigung ein Feststoff in einer Ausbeute von 35 % (1.72 g, 3.48 mmol) erhalten; roter Feststoff m.p. 183-184°C; IR (KBr): νₘₐₓ = 3100 (w), 3055 (w), 1642 (vs), 1447 (m), 1294 (s), 810 (m), 778 (s), 757 (m).

### 2. Allgemeine Durchführungsvorschrift der CBS-Reduktion der 1,1'-Diacylferrocene (B->C)

Unter Argon wird Oxazaborolidin (gemäß J. Am. Soc. 1987, 109, 5551-5553) (60 mol-%) in THF (5 mL) gelöst und auf 0 °C gekühlt. Es werden anschließend 2 eq. BH₃·SMe₂ in THF (5 mL) gelöst und 20% dieser Lsg. zur Katalysator-Lsg. hinzugegeben. Nach 5 min wird restliches BH₃·SMe₂ und eine Lösung des Diketons in THF (10 mL) gleichzeitig zur Katalysator-Lsg. gegeben. Nach 10 min Rühren bei 0 °C wird der Überschuß an BH₃·SMe₂ durch Zugabe von Methanol zerstört (2 mL). Anschließend wird die Mischung in ges. NH₄Cl-Lsg. (50 mL) gegeben und mit MTBE (3 x 25 mL) extrahiert. Die vereinigten org. Phasen werden mit Wasser (2 x 25 mL) und ges. NaCl-Lsg. (100 mL) gewaschen, getrocknet, aufkonzentriert und per Säulenchromatographie gereinigt.

*(*R,R*)-1,1'-Bis(α-hydroxyphenylmethyl)ferrocen:* Das entsprechende Diketon (11.82 g, 30.0 mmol) wird mit Oxazaborolidin (4.98 g, 18.0 mmol) und BH₃·SMe₂ (5.7 mL, 60.0 mmol) zur Reaktion gebracht. Nach Säulenchromatographie erhält man in 89 %iger Ausbeute (10.62 g, 26.4 mmol) das gewünschte Produkt *dl* : *meso* = 96 : 4. Die Kristallisation aus MTBE ergibt einen gelben Feststoff *dl* : *meso* = 98 : 2; *ee* > 99%; m.p. 130-132 °C; [α]_{D} = -74.3 (*c* = 0.97, Benzol); IR (KBr): νₘₐₓ = 3526 (vs), 3081 (w), 3026 (w), 1491 (m), 1452 (m), 1049 (m), 1017 (m), 828 (m), 721 (s), 699 (s).

*(*R,R*)-1,1'-Bis(α-hydroxy-*o*-tolylmethyl)ferrocen:* Das entsprechende Diketon (4.22 g, 10.0 mmol) wird mit Oxazaborolidin (1.66 g, 6.0 mmol) und BH₃·SMe₂ (1.90 mL, 20.0 mmol) reduziert und anschließend säulenchromatographisch gereinigt. Man erhält in 94 %iger Ausbeute einen gelben Feststoff (4.01 g, 9.4 mmol); *dl* : *meso* = 97 : 3; ee > 99%; m.p. 138 °C; [α]_{D} = -46.3 (*c* = 0.67, CHCl₃); IR (KBr): νₘₐₓ = 3270 (vs), 3077 (w), 2926 (w), 1043 (s), 820 (m), 738 (s).

*(*R,R*)-1,1'-Bis[α-hydroxy-(2-naphthyl)methyl]ferrocen:* Das entsprechende Diketon (996 mg, 2.00 mmol) wird mit Oxazaborolidin (332 mg, 1.20 mmol) und BH₃·SMe₂ (4.0 ml), 1M in THF) reduziert und anschließend per Säulenchromatographie gereinigt. Man erhält in 80 %iger Ausbeute einen gelben Feststoff (793 mg, 1.59 mmol); *dl* : *meso =* 97 : 3; ee > 99 %; m.p. 187-188 °C; [α]_{D} = +61.5 (c = 0.63, THF); IR (KBr): νₘₐₓ = 3380 (s), 3053 (w), 2863 (w), 1054 (m), 1017 (m), 786 (m), 751 (m).

### 3. Allgemeine Herstellung der Acetate (C->D)

Die Ferrocendiole werden in einer Mischung aus Pyridin/Acetanhydrid 2:1 gelöst und für 12 h bei RT belassen. Anschließend werden im Vakuum (0.7 mmHg, 5 h) verdampfbare Bestandteile abdestilliert. Die Produkte sind ausreichend rein und werden unverändert weiter eingesetzt.

*(*R,R*)-1,1'-Bis(α-acetoxyphenylmethyl)ferrocen:* Gelbes Öl *dl* : *meso* = 93 : 7; ee > 98 %; *α*]_{D} = -30.0 (*c* = 1.81, CHCl₃); IR (neat): νₘₐₓ = 3089 (w), 3066 (w), 3035 (m), 2937 (w), 1733 (vs), 1372 (s), 1241 (vs), 1019 (s), 830 (m), 731 (s), 700 (s).

*(*R,R*)-1,1'-Bis(α-acetoxy-*o*-tolylmethyl)ferrocen:* Dunkel braunes Öl d*l* : meso = 94 : 6; ee > 98 %; [α]_{D} = -57.7 (*c* = 0.96, CHCl₃); IR (neat): νₘₐₓ = 3025 (w), 2935 (s), 1720 (vs), 1450 (m), 1365 (m), 1225 (vs), 1020 (m), 820 (m), 740 (m).

*(*R,R*)-1,1'-Bis[α-acetoxy-(2-naphthyl)methyl]ferrocen:* Gelber Feststoff *dl* : *meso* = 84 : 16; m.p. 129-130 °C; [α]_{D} = -3.5 (*c* = 0.51, CHCl₃); IR (KBr): νₘₐₓ = 3054 (w) , 2957 (w), 1732 (vs), 1373 (m), 1233 (vs), 1043 (m), 1021 (m), 788 (m), 761 (m).

### 4. Allgemeine Arbeitsvorschrift zur Umsetzung der Ferrocenacetate mit Dimethylamin in THF / H₂O (D->E)

Die Ferrocenacetate werden in THF gelöst. Diese Lsg. wird mit einem 40 %igen Überschuß an wäßriger Dimethylamin-Lsg. versetzt. Nach Rühren für 12 h bei RT wird die Reaktionsmischung aufgearbeitet und durch Säulenchromatographie gereinigt.

*(*R,R*)-1,1'-Bis(*α-N,N-*dimethylaminophenylmethyl)ferrocen:* Das entsprechende Diacetat (7.95 g, 16.5 mmol) wird mit Dimethylamin (40 % in Wasser, 60 mL) in 40 mL THF / 10 mL Wasser umgesetzt. Man erhält das entsprechende Diamin als braunes Öl in 91% Ausbeute (6.79 g, 15.0 mmol); *dl* : *meso* = 96 : 4; [α]_{D} = +122.0 (c = 1.36, CHCl₃); IR (neat) : νₘₐₓ = 3060 (w), 3030 (w), 2950 (m), 2860 (w), 2810 (w), 2770 (s), 1455 (s), 1300 (m), 1005 (s), 830 (m), 740 (s), 700 (m).

*(*R,R*)-1,1'-Bis(α-*N,N*-dimethylamino-*o*-tolylmethyl)ferrocen*: Das entsprechende Diacetat (510 mg, 1.00 mmol) wird mit Dimethylamin (40 % in Wasser, 4 mL) in 10 mL THF / 2.5 mL Wasser vumgesetzt. Man erhält das entsprechende Diamin in 81 % Ausbeute als orangen Feststoff (389 mg, 0.81 mmol); *dl* : *meso* = 85 : 15; m.p. 104-106 °C; [α]_{D} = +120.1 (*c* = 1.29, CHCl₃); IR (KBr): νₘₐₓ = 3064 (w), 3020 (w), 1602 (w), 1006 (s), 823 (m), 739 (vs).

*(*R,R*)*-*1*,*1*'-*Bis*[α-N,N-*dimethylamino*-*(2-naphthyl)methyl]ferrocen:* Das entsprechende Diacetate (1.33 g, 2.28 mmol) wird mit Dimethylamin (40 % in Wasser, 20 mL) in 30 mL THF / 7.5 mL Wasser umgesetzt. Man erhält das entsprechende Diamin in 88 % Ausbeute als orangen Feststoff (1.10 g, 2.0 mmol); *dl* : meso = 92 : 8; m.p. 142-143 °C; [α]_{D} = -47.1 *(c* = 0.47, CHCl₃); IR (KBr): νₘₐₓ = 3058 (w), 2979 (w), 2944 (w), 2810 (m), 2762 (s), 1296 (m), 1011 (s), 828 (s), 762 (m).

### 5. Allgemeine Herstellvorschrift für die Synthese der Bromide (E->H)

Die entsprechenden Diamine werden in Et₂O (5 mL) gelöst, auf 0 °C gekühlt und mit Bu^{t}Li (*c* = 1.5 M; 3 eq) binnen 5 min versetzt. Die Lösung wird 30 min bei dieser Temp. gerührt. Anschließend wird eine Lsg. von 1,2-Dibromtetrachlorethan (3 eq) in Et₂O (5 mL) innerhalb von 10 min addiert. Die Mischung wird 3 h bei RT gerührt, aufgearbeitet und säulenchromatographisch gereinigt.

(αR,α'R)*-2,2'-Bis(*α-N,N-*dimethylaminophenylmethyl)-(*S,S*)-1,1*'-*dibromferrocen*: Das entsprechende Diamin (460 mg, 1.00 mmol) wird mit Bu^{t}Li (2.0 mL, 3.00 mmol) und anschließend mit (CBrCl₂)₂ (977 mg, 3.00 mmol) versetzt. Nach säulenchromatographischer Reinigung erhält man ein dunkel braunes Öl in 80 % Ausbeute (486 mg, 0.80 mmol), welches ein Diastereomer enthält (*ee* = 100 %). [α]_{D} = +154.5 (*c* = 0.88, CHCl₃); IR (neat): νₘₐₓ = 3084 (w), 3063 (w), 3026(w), 2816 (s), 2772 (s), 1601 (w), 1491 (w), 1009 (s), 756 (vs), 735 (vs).

*(*αR,α'R*)-2,2'-Bis(*α-N,N-*dimethylamino*-o-*tolylmethyl)-(*S,*S)-1,1*'-*dibromferrocen*: Das entsprechende Diamine (2.16 g, 4.49 mmol) wird mit Bu^{t}Li (8.9 mL, 13.48 mmol) und (CBrCl₂)₂ (4.39 g, 13.48 mmol) umgesetzt. Nach säulenchromatographischer Reinigung und Umkristallisation aus Diethylether / Hexan erhält man einen braunen Feststoff in 52 % Ausbeute (1.50 g, 2.35 mmol), welcher ein Diastereomer enthält (ee = 100 %). mp: 169-171 °C; [α]_{D} = +224.2 (*c* = 0.78, CHCl₃); IR (KBr): νₘₐₓ = 3071 (w), 3046 (w), 3021 (w), 1601 (w), 823 (s), 744 (s).

*(*αR,α'R*)-2,2'-Bis(*α-N,N-*dimethylamino-2-naphthylmethyl)-(*S,S*)-1,1'-dibromferrocen:* Das entsprechende Diamine (0.60 g, 1.09 mmol) wird mit Bu^{t}Li (2.2 mL, 3.26 mmol) und (CBrCl₂)₂ (1.06 g, 3.26 mmol) umgesetzt. Nach säulenchromatographischer Aufreinigung und Umkristallisation aus Diethylether / Hexan erhält man einen braunen Feststoff in 43 % Ausbeute (0.33 g, 0.47 mmol), welcher ein Diastereomer enthält (*ee >* 98 %). mp: 147-148 °C; [α]_{D} = -49.6 (*c* = 0.74, CHCl₃); IR (KBr) : νₘₐₓ = 3057 (w), 1601 (w), 1508 (w), 907 (s), 824 (s), 735 (s).

### 6. Allgemeine Herstellvorschrift für die Dibromferrocene (H->I)

Die entsprechenden Aminobromide (1 mmol) werden in Acetanhydrid (4 mL) gelöst und auf 100 °C für 2.5 h erhitzt. Anschließend werden die flüchtigen Bestandteile im Vakuum (0.7 mmHg, 3 h) entfernt. Man erhält in quantitativer Ausbeute die entsprechenden Acetate (> 95%ig, NMR).

*(α*R,*α*'R*)-2,2'-Bis(α-acetoxyphenylmethyl)-(*S,S*)-1,1'dibromferrocen:* Gelber Feststoff mp:145-147 °C; [α]_{D} = +83.2 *(c* = 0.90, CHCl₃); IR (KBr): νₘₐₓ = 1738 (vs), 1225 (vs).

*(α*R,*α*'R*)-2,2'-Bis(α-acetoxy-*o*-tolylmethyl)-(*S,S*)-1,1'dibromferrocen:* Gelber Feststoff mp: 143-144 °C; [α]_{D} = +71.5 *(c* = 0.92, CHCl₃); IR (KBr) 1735 (vs), 1231 (vs).

*(α*R,*α*'R*)-2*,*2*'*-Bis(α-acetoxy-2-naphthylmethyl)-(*S,S*)-1*,*1*'*dibromferrocen:* Gelber Feststoff mp: 90-93 °C; [α]_{D} = +55.6 (c = 1.11, CHCl₃); IR (KBr): νₘₐₓ = 3057 (w), 3025 (w), 1748 (vs), 1235 (vs).

### 7. Allgemeine Durchführungsvorschrift für die Reaktion der Acetate mit Organozinkreagentien (I->J)

Zu einer Lsg. der entsprechenden Acetate in 5 mL trockenem THF werden bei -78 °C unter Argon die Organozinkreagenzien (3 eq) und BF₃·OEt₂ (2 eq) gegeben. Die Reaktionsmischung wird binnen 1.5 h auf RT aufgewärmt und anschließend nach einer weiteren Stunde wie gehabt aufgearbeitet. Das Rohprodukt wird per Säulenchromatographie gereinigt.

*(*αR,α'R*)-2,2'-Bis(α-methylphenylmethyl)-(*S,S*)-1,1'dibromferrocen:* Das Acetat (219 mg, 0.34 mmol) wird mit BF₃·OEt₂ (84 µL, 0.68 mmol) und Dimethylzink (neat; 1.03 mmol, 71 µL) versetzt. Man erhält in 98 % Ausbeute ein braunes Öl (184 mg, 0.33 mmol); ee = 100 %; [α]_{D} = +171.4 (*c* = 1. 10, CHCl₃); IR (neat): νₘₐₓ = 3084 (w), 3061 (w), 3028 (w), 1601 (w), 1584 (w), 1493 (s), 816 (s), 772 (vs), 706 (vs).

*(*αR,α'R*)-2,2'-Bis[α-methyl-(*o*-tolyl)methyl]-(*S,S*)-1,1'dibromferrocen:* Das Acetat (134 mg, 0.20 mmol) wird mit BF₃·OEt₂ (49 µL, 0.40 mmol) und Dimethylzink (neat; 0.60 .mmol, 41 µL) versetzt. Nach Säulenchromatographie erhält man in 100 % Ausbeute ein braunen Feststoff (116 mg, 0.20 mmol); *ee* = 100 %; mp = 76-78 °C; [α]_{D} = +118.9 (*c* = 0.73, CHCl₃); IR (KBr): νₘₐₓ = 3065 (w), 3021 (w), 1603 (w), 1491 (w), 816 (s), 758 (s).

*(α*R,*α'*R*)-2,2'-Bis[α-methyl-2-naphthylmethyl)-(*S,S*)-1,1'dibromferrocen:* Das Acetat (195 mg, 0.26 mmol) wird mit BF₃·OEt₂ (65 µL, 0.52 mmol) und Dimethylzink (neat; 0.79 mmol, 54 µL) versetzt. Nach säulenchromatograhischer Aufreinigung erhält man in 92 % Ausbeute einen gelben Feststoff (55 mg, 0.24 mmol); *ee* = 100 %; mp = 58-60 °C; [α]_{D} = +86.5 (*c* = 1.04, CHCl₃); IR (KBr): νₘₐₓ = 3055 (w), 3021 (w), 1601 (w), 1508 (w), 820 (s), 750 (s), 733 (s).

### 8. Synthese der C₂-symmetrischen Diphosphine (J->L)

Zu einer Lösung aus THF (5 mL) und dem entsprechenden Bromid wird bei -78°C BuⁿLi (*c* = 1.50; 3 eq) gegeben und anschließend die Lsg. nach 15 min mit Diphenylchlorophosphin (neat; 4 eq) versetzt. Die Reaktionsmischung wird auf RT erwärmt und 1 h bei dieser Temp. gerührt, bevor sie nach der üblichen Prozedur säulenchromatographisch aufgearbeitet wird. Nach Umkristallisation aus Diethylether erhält man die C_{*2*}symmetrischen Diphosphine.

*(α*R*,α'*R*)-2,2'-Bis(α-methylphenymethyl)-(*S,S*)-1,1'bis(diphenylphosphino)ferrocen:* Das entsprechende Bromid (276 mg, 0.50 mmol) wird mit BuⁿLi (1.00 mL, 1.50 mmol) und ClPPh₂ (360 µL, 2.00 mmol) umgesetzt. Nach Säulenchromatographie und Umkristallisation aus Diethylether erhält man einen orangen Feststoff in 68 % Ausbeute (263 mg, 0.34 mmol); mp:181-182 °C; [α]_{D} = -245.2 (*c* = 0.40, CHCl₃); IR (KBr) : νₘₐₓ = 3056 (w), 3026 (w) , 1600 (w), 1583 (w), 1493 (w), 748 (s), 741 (s), 697 (vs).

*(*αR,α'R*)-2,2'-Bis[α-methyl-(*o*-tolyl)methyl]-(*S,S*)-1,1'bis(diphenylphosphino)*-*ferrocen:* Das entsprechende Bromid (100 mg, 0.17 mmol) wird mit BuⁿLi (345 µL, 0.52 mmol) und ClPPh₂ (120 µL, 0.68 mmol) umgesetzt. Nach Säulenchromatographie und Umkristallisation aus Diethylether erhält man einen orangen Feststoff in 64 % Ausbeute (85 mg, 0.11 mmol); mp:164-166 °C; [α]_{D} = -402.4 (*c* = 0.67, CHCl₃); IR (KBr): νₘₐₓ = 3054 (w), 3019 (w), 1603 (w), 1586 (w), 1570 (w), 1489 (m), 735 (vs), 698 (vs).

*(*αR,α'R*)-2,2'-Bis[α-methyl-2-naphthylmethyl]-(*S,S*)-1,1'bis(diphenylphosphino)-ferrocen:* Das entsprechende Bromide (300 mg, 0.46 mmol) wird mit BuⁿLi (1.12 mL, 1.68 mmol) und ClPPh₂ (330 µL, 1.84 mmol) umgesetzt. Nach Säulenchromatographie und Umkristallisation aus Diethylether / Dichloromethan erhält man einen orangen Feststoff in 46 % Ausbeute (184 mg, 0.21 mmol); mp: 208-210 °C; [α]_{D} = -256.3 (*c* = 0.54, CHCl₃); IR (KBr): νₘₐₓ = 3052 (w), 1601 (w), 1584 (w), 822 (s), 741 (s), 689 (s).

### 9. Synthese der planarchiralen Diphosphinoferrocene ausgehend vom Acetat (I->G)

Das entsprechende Acetat (174 mg, 0.27 mmol) wird in 5 mL THF gelöst und auf 0 °C gekühlt. Alsdann wird BF₃·OEt₂ (2.2 eq, 73 µL, 0.59 mmol) tropfenweise zu der Mischung gegeben. Nach 15 min wird eine 1 M Lsg. aus LiHBEt₃ in THF (2.2 eq, 0.59 mmol, 0.59 mL) hinzugegeben und die Mischung bei RT für 3 h belassen. Nach normaler Aufarbeitung und Reinigung durch Säulenchromatographie erhält man das entsprechende Dibromid in 66 % Ausbeute (93 mg, 0.18 mmol) als gelben Feststoff.

*(*S*,*S*)-2,2'-Dibenzyl-1,1'-dibromoferrocen*: mp: 61-64 °C; [α]_{D} = +81.8 (*c* = 1.05, CHCl₃); IR (KBr) : νₘₐₓ = 3083 (w), 3059 (w), 3025 (w), 1601 (w), 1582 (w), 814 (m), 724 (m), 707 (s), 693 (s).

*(*S,S*)-2,2'-Dibenzyl-1,1'-bis(diphenylphosphino)ferrocen*: Das Dibromid kann analog der oben genannten Vorschrift (J->L) in das Diphosphin überführt werden. Dazu wird das Bromid (135 mg, 0.26 mmol) mit BuⁿLi (510 µL, 0.77 mmol) und ClPPh₂ (185 µL, 1.03 mmol) umgesetzt. Nach Säulenchromatographie erhält man einen gelben Feststoff in 66 % Ausbeute (127 mg, 0.17 mmol); mp: 66-68 °C; [α]_{D} = - 329.3 (*c* = 1.60, CHCl₃); IR (KBr): νₘₐₓ = 3028 (m), 3001 (m), 1660 (m), 1601 (s), 1586 (s), 1570 (m), 741 (vs), 696 (vs).

### 10. Synthese der Diaminodiphosphinferrocene (E->F)

Unter Befolgung der Vorschrift 5 werden die entsprechenden Diamine (2.40 g, 5.3 mmol) mit Bu^{t}Li (10.6 mL, 15.9 mmol) und ClPPh₂ (3.8 mL, 21.2 mmol) umgesetzt. Nach säulenchromatographischer Aufreinigung erhält man einen gelben Feststoff in 49 % Ausbeute (2.14 g, 2.6 mmol), welcher ein Diastereomer enthält (*ee* > 99 %).

(*α*R*,α'*R)-*2,2'*-*Bis(α*-N,N-*dimethylaminophenymethyl*)-*(*S,S*)-1,1'-bis(diphenylphosphino)-ferrocen:* mp: 245-246 °C; [α]_{D} = -330.3° (*c* = 1.00, CHCl₃); IR (KBr): νₘₐₓ = 3090 (w), 3064 (w), 3030 (w), 2951 (m), 2856 (w), 2811 (m), 2764 (s), 1450 (s), 1006 (s), 814 (m), 737 (s), 703 (s) cm⁻¹.

### 11. Allgemeine Herstellvorschrift für die Synthese der Diacetatdiphosphinferrocen (F -> M)

Unter Befolgung der Vorschrift 6 werden die entsprechenden Diaminodiphosphinferrocene (1 mmol) in Acetanhydrid (4 mL) gelöst und auf 100 °C für 2.5 h erhitzt. Anschließend werden die flüchtigen Bestandteile im Vakuum (0.7 mmHg, 3 h) entfernt. Man erhält in quantitativer Ausbeute einen gelben Feststoff (> 95%ig, NMR) welcher ein Diastereomer enthält (ee > 99 %).

*(a*R,*a'*R*)-2,2'-Bis(a-Acetoxyphenylmethyl)-(*S,S*)-1,1'bis(diphenylphosphino)ferrocen:* mp: 184 °C (Zersetzung); [a]_{D} = -169.6 (*c* = 0.46, CHCl₃);

### 12. Allgemeine Arbeitsvorschrift zur Umsetzung der Diaminodiphosphinferrocene mit verschiedenen Aminen in CH₃CN / H₂O (M -> N)

Die Diacetatdiphosphinferrocen wird in CH₃CN gelöst. Diese Lsg. wird mit 50 Äquivalenten der entsprechenden Amine versetzt und auf 90 °C für 12 h erhitzt. Die Reaktionsmischung wird aufgearbeitet und durch Säulenchromatographie gereinigt.

*(a*R,*a'*R*)-2,2'-Bis(a-Pyrrolidinphenylmethyl) - (*S,S*)-1,1'bis(diphenylphosphino)-ferrocen:* Das entsprechende Diacetat (0.30 g, 0.35 mmol) wird mit Pyrrolidin (1.46 mL, 17.5 mmol) in 2 mL CH₃CN/0.2 mL Wasser umgesetzt. Man erhält das entsprechende Diamin als orangen Feststoff in 65 % Ausbeute nach säulenchromatographischer Aufreinigung und Umkristallisation aus Diethylether / Hexan: mp: 242 °C (Zersetzung); [a]_{D} = -317.5 (*c* = 0.53, CHCl₃); IR (KBr): : nₘₐₓ = 3067 (w), 3024 (w), 1601 (w), 1585 (w), 737 (s), 698 (s).

*(a*R,*a*'R*)-2*,*2*'*-Bis[a-(*N*-Methyl-*N*-cyclohexylamino)-phenylmethyl]-(*S,S*)-1,1'-bis(diphenylphosphino)ferrocen:* Das entsprechende Diacetat (0.16 g, 0.18 mmol) wird mit *N*-Methyl-*N*-cyclohexylamin (1.20 mL, 9.3 mmol) in 2 mL CH₃CN / 0.2 mL Wasser umgesetzt. Man erhält das entsprechende Diamin als gelben Feststoff in 87 % Ausbeute nach säulenchromatographischer Aufreinigung und Umkristallisation aus Hexan: mp: 224 °C (Zersetzung); [a]_{D} = -290.2 (*c* = 0.57, CHCl₃); IR (KBr) : nₘₐₓ = 3071 (w), 3053 (w), 3001 (w), 1600 (w), 1584 (w), 741 (s), 701 (s).

### 13. Hydrierung der (Z)-Methyl-β-(2-naphthyl)-α-acetamidoacrylate

In einem trockenen 50 mL Schlenkgefäß werden unter Argon [Rh(nbd)₂]BF₄-Komplex (3.7 mg, 0.01 mol) plaziert, und der entsprechende Ligand (0.01 mol) in 8 mL MeOH (HPLC-Reinheit o. technischer Qualität) gelöst addiert. Binnen 15 bis 30 min hat sich das Diphosphin gelöst. Eine Lsg. aus 2 mL MeOH und (*Z*)-Methyl-β-(2-naphthyl)-α-acetamidoacrylat (0.269 g, 1 mmol) wird hinzugegeben und anschließend ein Ballon mit H₂ gefüllt (*ca* 1.0 bar) an das System angeschlossen. Nach Spülen des Systems mit H₂ wird der H₂-Ballon nach Rühren für weitere 10 min entfernt und das MeOH im Vakkum abgezogen. Der Rückstand wird an Kieselgel säulenchromatographisch gereinigt. Man erhält das gewünschte Produkt in quantitativer Ausbeute.

*(*R*)-Methyl-3-(2-naphthyl)-2-acylamidopropanoat:* Öl; [α]_{D} = -104.3 (*c* = 0.92, CHCl₃; ee = 99.4 %) [Lit: +97.8 (*c* = 1, CHCl₃)]; Die spektroskopischen Daten stimmen mit denen aus der Literatur überein (J. Am. Chem. Soc. 1993, 115, 10125-10138).

### 14. Hydrierung von (Z)-β-(2-Naphthyl)-α-acetamidoacrylsäure (übliches Verfahren)

Man verfährt wie für die entsprechenden Ester beschrieben. Die entsprechende Säure (0.255 g, 1 mmol) wird 10 min hydriert. Anschließend wird das MeOH im Vakuum entfernt und 5 mL trockener Ether sowie 5 mL trockenes MeOH zum Rückstand addiert. Dann werden 2 Äquivalente von Me₃SiCHN₂ (*c* = 2 M; 1.0 mL, 2.0 mmol) tropfenweise zur Reaktionsmischung gegeben. Nach 1h wird das Lsgm. im Vakuum entfernt und der Rückstand wird wie im oben beschriebenen Fall behandelt und analysiert.

*(*R*)-Methyl-3-(2-naphthyl)-2-acylamidopropanoate:* Öl; [α]_{D} = -104.3 (*c* = 0.92, CHCl₃; ee = 98.2 %) [Lit: +97.8 (*c* = 1, CHCl₃)]; Die spektroskopischen Daten stimmen mit denen aus der Literatur überein (J. Am. Chem. Soc. 1993, 115, 10125-10138).

## Patentansprüche

1. Verwendung der enantiomerenangereicherten Liganden der allgemeinen Formel I sowie deren Salze 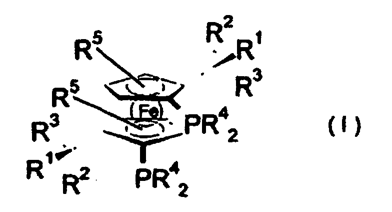 worin
R¹, R² unabhängig voneinander bedeuten H, N(C₁-C₈)-Alkyl₂, NH(C₁-C₈)-Acyl, N(C₁-C₈)-Acyl₂, O(C₁-C₈)-Acyl, (C₁-C₈)-Alkyl, (C₁-C₈)-Alkoxy, (C₂-C₈)-Alkoxyalkyl, welche gegebenenfalls linear oder verzweigt vorliegen, (C₃-C₇)-Cycloalkyl (C₆-C₁₈)-Aryl, wie Phenyl; 1-, 2-Naphthyl oder Anthryl,
R³ bedeutet (C₃-C₇)-Cycloalkyl, (C₆-C₁₈)-Aryl, wie Phenyl, 1-, 2-Naphthyl oder Anthryl, wobei die eben genannten Reste gegebenenfalls einfach oder mehrfach mit linear oder verzweigten (C₁-C₈)-Alkyl, mit Halogenen substituiert sein können,
R⁴ bedeutet (C₁-C₈)-Alkyl, (C₃-C₇)-Cycloalkyl, (C₆-C₁₈)-Aryl, wie Phenyl, 1-, 2-Naphthyl oder Anthryl, wobei die eben genannten Reste gegebenenfalls einfach oder mehrfach mit linear oder verzweigten (C₁-C₈)-Alkyl, mit Halogenen substituiert sein können,
R⁵ bedeutet H,
Zur homogenen Katalytischen enantioselektiven Hydrierung von C=C- bzw. C=N-Doppelbindungen.

2. Verwendung der Liganden nach Anspruch 1,
**dadurch gekennzeichnet, daß**
R¹, R² unabhängig voneinander bedeuten H, O(C₁-C₈)-Acyl, N(C₁-C₈)-Alkyl₂, (C₁-C₈)-Alkyl
R³ bedeutet (C₆-C₁₈)-Aryl, wie Phenyl, 1-,2-Naphthyl oder Anthryl, wobei die eben genannten Reste gegebenenfalls einfach oder mehrfach mit linear oder verzweigten (C₁-C₈)-Alkyl, mit Halogenen substituiert sein können,
R⁴ bedeutet Phenyl,
R⁵ bedeutet H.

3. Verwendung der enantiomerenangereicherten Komplexe der allgemeinen Formel II und deren Salze 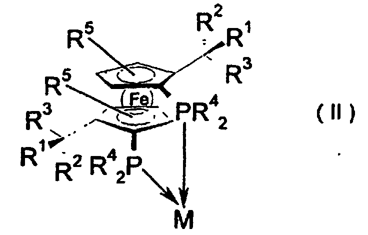 worin R¹ bis R⁵ die in Anspruch 1 angegebene Bedeutung innehaben und M ein Metallatom oder -ion der Nebengruppe 8, wie zB. Co, Ni, Rh, Ru, Ir, Pd, Re oder Pt ist, zur homogenen katalytischen enantioselektiven Hydrierung von C=C- bzw. C=N-Doppelbindungen.

4. Verwendung der Komplexe nach Anspruch 3,
**dadurch gekennzeichnet, daß**
R¹ bis R⁵ die in Anspruch 2 angegebene Bedeutung besitzen.

5. Verwendung der Komplexe nach Anspruch 4,
**dadurch gekennzeichnet, daß**
R¹ bis R⁵ die in Anspruch 3 angegebene Bedeutung besitzen.

## Claims

1. Use of enantiomer-concentrated ligands having the general formula I and salts thereof 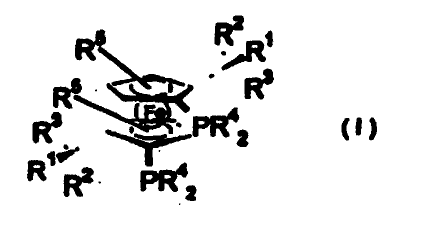 wherein
R¹, R² mutually independently denote H, N(C₁-C₈) alkyl₂, NH(C₁-C₈) acyl, N(C₁-C₈) acyl₂, O(C₁-C₈) acyl, (C₁-C₈) alkyl, (C₁-C₈) alkoxy, (C₂-C₈) alkoxyalkyl, which are optionally linear or branched, (C₃-C₇) cycloalkyl (C₆-C₁₈) aryl, such as phenyl; 1-, 2-naphthyl or anthryl,
R³ denotes (C₃-C₇) cycloalkyl, (C₆-C₁₈) aryl, such as phenyl, 1-, 2-naphthyl or anthryl, whereby the cited radicals can optionally be monosubstituted or polysubstituted with linear or branched (C₁-C₈) alkyl, with halogens,
R⁴ denotes (C₁-C₈) alkyl, (C₃-C₇) cycloalkyl, (C₆-C₁₈) aryl, such as phenyl, 1-, 2-naphthyl or anthryl, whereby the cited radicals can optionally be monosubstituted or polysubstituted with linear or branched (C₁-C₈) alkyl, with halogens,
R⁵ denotes H,
for the homogeneous catalytic enantioselective hydrogenation of C=C or C=N double bonds.

2. Use of the ligands according to claim 1, **characterised in that**
R¹, R² mutually independently denote H, O(C₁-C₈) acyl, N(C₁-C₈) alkyl₂, (C₁-C₈) alkyl
R³ denotes (C₆-C₁₈) aryl, such as phenyl, 1-, 2-naphthyl or anthryl, whereby the cited radicals can optionally be monosubstituted or polysubstituted with linear or branched (C₁-C₈) alkyl, with halogens,
R⁴ denotes phenyl,
R⁵ denotes H.

3. Use of enantiomer-concentrated complexes having the general formula II and salts thereof 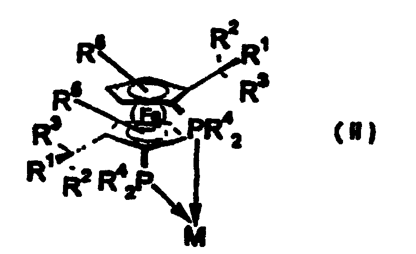 wherein R¹ to R⁵ have the meaning stated in claim 1 and M is a metal atom or ion from subgroup 8, such as e.g. Co, Ni, Rh, Ru, Ir, Pd, Re or Pt, for the homogeneous catalytic enantioselective hydrogenation of C=C or C=N double bonds.

4. Use of the complexes according to claim 3, **characterised in that**
R¹ to R⁵ have the meaning stated in claim 2.

5. Use of the complexes according to claim 4, **characterised in that**
R¹ to R⁵ have the meaning stated in claim 3.

## Revendications

1. Utilisation des ligands enrichis en énantiomères de formule générale I ainsi que de leurs sels 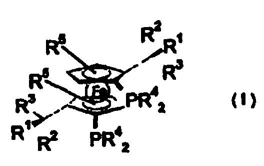 où
R¹, R² représentent indépendamment l'un de l'autre H, un N-alkyle en C₁ à C₈)₂, un NH-acyle en C₁ à C₈, un N-(acyle en C₁ à C₈)₂, un O-acyle en C₁ à C₈, un alkyle en C₁ à C₈, un alcoxy en C₁ à C₈, un alcoxyalkyle en C₂ à C₈, qui le cas échéant peut se présenter sous forme linéaire ou ramifiée, un cycloalkyle en C₃ à C₇, un aryle en C₆ à C₁₈, comme un phényle ; un 1- ou 2-naphtyle ou un anthryle,
R³ représente un cycloalkyle en C₃ à C₇, un aryle en C₆ à C₁₈, comme un phényle, un 1- ou 2-naphtyle ou un anthryle, où les radicaux qui viennent d'être mentionnés peuvent être éventuellement mono- ou polysubstitués par un alkyle en C₁ à C₈ linéaire ou ramifié ou par des halogènes,
R⁴ représente un alkyle en C₁ à C₈, un cycloalkyle en C₃ à C₇, un aryle en C₆ à C₁₈, comme un phényle, un 1- ou 2-naphtyle ou un anthryle, les radicaux qui viennent d'être mentionnés pouvant être éventuellement mono- ou polysubstitués par des alkyles en C₁ à C₈ linéaires ou ramifiés ou par des halogènes,
R⁵ représente H,
pour l'hydrogénation énantiosélective à catalyse homogène de doubles liaisons C=C ou selon les cas C=N.

2. Utilisation des ligands selon la revendication 1,
**caractérisée en ce que**
R¹, R² représentent indépendamment l'un de l'autre H, un O-acyle en C₁ à C₈, un N-(alkyle en C₁ à C₈)₂, un alkyle en C₁ à C₈,
R³ représente un aryle en C₆ à C₁₈, comme un phényle, un 1- ou 2-naphtyle ou un anthryle, où les radicaux qui viennent d'être mentionnés peuvent le cas échéant être mono- ou polysubstitués par de alkyles en C₁ à C₈ linéaires ou ramifiés ou par des halogènes,
R⁴ représente un phényle,
R⁵ représente H.

3. Utilisation des complexes enrichis en énantiomères de formule générale II et de leurs sels 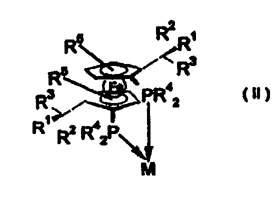 où
R¹ à R⁵ ont la signification donnée dans la revendication 1 et M est un atome ou ion métallique du sous-groupe 8, comme par exemple Co, Ni, Rh, Ru, Ir, Re ou Pt, pour l'hydrogénation énantiosélective à catalyse homogène de doubles liaisons C=C ou selon les cas C=N.

4. Utilisation des complexes selon la revendication 3,
**caractérisée en ce que**
R¹ à R⁵ ont la signification donnée dans la revendication 2.

5. Utilisation des complexes selon la revendication 4,
**caractérisée en ce que**
R¹ à R⁵ ont la signification indiquée dans la revendication 3.
